Europäisches Patentamt

European Patent Office

Office européen des brevets

(19)

(11) Publication number: **0 105 749**
**A2**

(12) **EUROPEAN PATENT APPLICATION**

(21) Application number: **83305950.4**

(22) Date of filing: **30.09.83**

(51) Int. Cl.³: **A 61 K 35/12**
**C 07 G 17/00, C 12 P 1/00**
**//C12R1/91**

(30) Priority: **01.10.82 GB 8228144**

(43) Date of publication of application:
**18.04.84 Bulletin 84/16**

(84) Designated Contracting States:
**AT BE CH DE FR GB IT LI LU NL SE**

(71) Applicant: **BIO-COM Inc.**
**Apartado 850 Calle Aquilino de la Guardia No. 8**
**Panama 1(PA)**

(72) Inventor: **Adamopoulos, Dimitri**
**Spartis 4 Strofyli-Kifissia**
**Attiki(GR)**

(72) Inventor: **Pascuchi, Josep Maria Vich I**
**L Grifols C/Jesus/Maria 6**
**Barcelona-22(ES)**

(74) Representative: **Woods, Geoffrey Corlett et al,**
**J.A. KEMP & CO. 14 South Square Gray's Inn**
**London WC1R 5EU(GB)**

(54) Compositions for use in inducing immunosuppression or immunoregulation of the immune response to an antigen.

(57) A composition, suitable for use in suppressing the immune response of a human or animal to an antigen, comprises a suppressor factor which is capable of inducing immunosuppression or immunoregulation of the immune response to an antigen in a human or animal but comprises substantially no inducer factor capable of inducing the ability to mount a cell-mediated immune response in a human or animal to any antigen or to an antigen to which the suppressor factor is specific, said suppressor factor having been derived from cells of lymphoid origin of a human or other mammal and the composition containing substantially no molecules from said cells of lymphoid origin with a molecular weight of 12,000 or more.

EP 0 105 749 A2

- 1 -

"COMPOSITIONS FOR USE IN INDUCING IMMUNOSUPPRESSION OR
IMMUNOREGULATION OF THE IMMUNE RESPONSE TO AN ANTIGEN"

This invention relates to compositions for use in inducing immunosuppression or immunoregulation of the immune response to an antigen and, for example, can be used in the treatment of allergic disorders and in the prevention of transplant rejection.

Allergic disorders are usually resistant to existing treatments, e.g. antihistaminic drugs, specific desensitization, corticoid therapy and gamma globulin injections. They are a major problem and they constitute a severe handicap for the patient.

Pollen is in general responsible for allergic disorders such as asthma, rhinitis, conjunctivitis. These manifestations are seasonal depending on the presence of pollen in the air. Similarly house dust may create the same type of problems in a perennial fashion.

It is known that dialysates obtained from lymphocytes contain an inducer factor which, on administration, is capable of inducing in a patient the ability to mount a cell-mediated immune response to an antigen. Inducer factor is at present thought to be an informational factor which transfers its information to T-helper cells.

It has now been found that cell fractions obtained from cells of lymphoid origin and which contain no inducer factor in fact contain a suppressor factor. This suppressor factor is capable of inducing immunosuppression or immunoregulation of the immune response to an antigen in a subject. The suppressor factor can therefore induce a state of immunological tolerance to an antigen. It has been found that the suppressor factor can be used to suppress the immune response of a human or animal to an antigen and may be employed to treat allergic disorders and to prevent transplant rejection.

- 2 -

Accordingly, the present invention provides a composition, suitable for use in suppressing the immune response of a human or animal to an antigen, which composition comprises a suppressor factor which is capable of inducing immunosuppression or immunoregulation of the immune response to an antigen in a human or animal but comprises substantially no inducer factor capable of inducing the ability to mount a cell-mediated immune response in a human or animal to any antigen or to an antigen to which the suppressor factor is specific, said suppressor factor having been derived from cells of lymphoid origin of a human or other mammal and the composition containing substantially no molecules from said cells of lymphoid origin with a molecular weight of 12,000 or more.

A composition according to the present invention produces an increase in migration, as measured by the Migration Index (M.I.) in the Leucocyte Migration Inhibition (L.M.I.), of at least 20% when compared with the M.I. when the L.M.I. test is effected without incubation of the leucocytes used in the test with the present composition. Preferably, a composition according to the invention has an M.I. of 1 or more when assayed in the L.M.I. test. Inducer factor decreases the M.I. in the L.M.I. test. The M.I. is considered statistically positive when lower than 0.8 in relation to inducer factor. The L.M.I. test is a well-known test in the art and is explained in more detail hereinafter.

The suppressor factor is preferably specific to a known antigen, in which case the composition of the invention contains substantially no inducer factor specific to that antigen. In order to obtain suppressor factor specific to a given antigen the suppressor factor can be obtained from cells from a donor sensitised to a given antigen, for example by immunisation.

- 3 -

Alternatively, a composition of the invention may comprise suppressor factor which is antigen non-specific, having been obtained from cells of lymphoid origin extracted from a human or other mammal which has not been immunised with respect to a particular antigen. Non-specific fractions can be derived from T-suppressor cells from non-selected blood donors or non-immunised animals.

The antigen-specific suppressor factor has a clear specificity and is capable of suppressing the antigen-elicited response in sensitized animals _in vivo_ or the _in vitro_ response of immune lymphocytes to a given antigen. The non-specific suppressor factor also shows suppressive activity _in vivo_ and _in vitro_ against various antigens. Without wishing to be bound by theory, it is currently believed that antigen-specific suppressor factor is an informational factor because of its ability to transfer immunological information concerning a specific antigen and that non-specific suppressor factor is a stimulating or nutritional factor which stimulates all T-suppressor cells of a patient.

According to the present invention, compositions according to the invention may be prepared from cells of lymphoid origin derived from a human or other mammal. The cells of lymphoid origin preferably have been extracted from a human or other mammal, and optionally cultured _in vitro_, or have been obtained by culturing cells of an established cell line of lymphoid origin _in vitro_, for example cells of a lymphoblastoid cell line, in the presence of a composition of the invention. In the latter case, the composition of the invention will preferably have been prepared from cells of lymphoid origin extracted from a human or other mammal and optionally cultured _in vitro_.

- 4 -

The cells of lymphoid origin may be obtained by drawing blood or removing the spleen or lymph nodes from a mammal. The cells thus may be lymphocytes such as peripheral blood lymphocytes or cells from lymphatic tissues such as the spleen or lymph nodes. The peripheral blood lymphocytes may be obtained by leucopheresis or by repeated bleedings, i.e. bleeding a mammal every two or three months, for example. It may be necessary to kill a mammal to obtain cells. Alternatively, the blood from human blood donors may be pooled and used as the starting point for the preparation of a composition according to the present invention comprising suppressor factor.

Suppressor factor specific to a given antigen, for example pollen, house dust, or transplantation or viral antigens, can be prepared by immunizing a human or other mammal with respect to a particular antigen prior to extracting the cells of lymphoid origin from that human or mammal. Any suitable mammal, for example guinea pigs, mice, calves or humans, can be used. Preferably the mammal is healthy. To obtain suppressor factor specific to a particular antigen immunization is generally achieved by injecting the antigen into a mammal. Typically several injections over a period of time are required. The point at which the mammal has acquired the necessary degree of immunization can be evaluated by, for example, a skin test, leucocyte migration inhibition test and/or lymphocyte transformation test. Non-specific suppressor factor cell fractions can be produced from a non-immunized or immunized mammal.

A composition according to the invention can be prepared by obtaining from cells of lymphoid origin extracted from a human or other mammal the cell fraction containing substantially no molecules with a molecular weight of 12,000 or more. If desired or appropriate, the lymphocytes may be first separated prior to obtaining the desired cell fraction. The cell fraction may be obtained by dialysis or ultrafiltration of the disrupted cells in conventional manner. The cells may be disrupted by freezing-thawing repeatedly or by means of a homogeniser or sonication. Dialysis can be effected using a dialysis membrane with an exclusion size of 12,000 (molecular weight), for example Visking cellulose. Ultrafiltration may be conducted through an Amicon filter with an exclusion size of 12,000 (molecular weight).

Once a cell fraction containing substantially no molecules with a molecular weight of 12,000 or more has been prepared, a composition according to the invention can be obtained in one of several ways. First, the inducer factor can be removed from the cell fraction by immunoadsorption e.g. affinity chromatography. In order to obtain a composition comprising a suppressor factor specific to a particular antigen where the cells of lymphoid origin have been obtained from a human or other mammal immunised with respect to that antigen, the inducer factor specific to that antigen is removed from the cell fraction.

Inducer factor specific to a particular antigen binds to that antigen. Consequently, a cell fraction comprising suppressor and inducer factor specific to a given antigen can be passed over a solid-phase support to which the antigen in question is bound. For example, the cell fraction may be passed through a column containing gel to which the antigen is bound. The inducer factor binds to the antigen. The cell fraction thus obtained contains substantially no inducer factor specific to that antigen.

The second manner in which a composition according to the invention may be obtained is to purify the suppressor factor from the cell fraction by immunoadsorption or affinity chromatography. A suppressor factor specific to a given antigen binds to gamma-globulin specific to that antigen. The cell fraction containing both suppressor and inducer factor specific to a given antigen can be passed over a solid-phase support to which gamma-globulin specific to that antigen is bound. The suppressor factor binds to the gamma-globulin. The suppressor factor can then be recovered from solid-phase support, for example by treatment with glycine-HCl or 4M MgSO$_4$. The recovered suppressor factor is a composition according to the invention.

Third, the suppressor factor can be obtained by column chromatography in which the components of the cell fraction containing substantially no molecules with a molecular weight of 12,000 or more are separated on the basis of molecular weight. The cell fraction can be passed through a suitable column such as Sephadex G-10 or G-25 or Biogel P-10 and the fraction or fractions containing suppressor factor collected.

An alternative method for the preparation of a composition according to the present invention is to separate T-suppressor cells from other cells of lymphoid origin obtained from a mammal and prepare from the T-suppressor cells, for example by dialysis or ultra-filtration, a cell fraction containing substantially no molecules with a molecular weight of 12,000 or more. The T-suppressor cells contain practically no inducer factor. It is thus compositions according to the present invention which are derived from T-suppressor cells which contain substantially no inducer factor capable of inducing the ability to mount a cell-mediated immune response in a human or animal to any antigen.

When adopting this method, it is possible to draw blood from a mammal, separate the T-suppressor cells in the plasma and dialyse or subject to ultrafiltration disrupted T-suppressor cells to obtain the desired cell fraction. Plasmapheresis can be used. As before, the cells of lymphoid origin may be lymphocytes such as peripheral blood lymphocytes or cells from lymphatic tissues such as the spleen or the lymph nodes. It is also possible to obtain suppressor factor from the cells of a T-suppressor cell line or from the culture medium in which the cells are grown.

The cells of lymphoid origin obtained from a mammal are preferably incubated at $45°C$ for 1-2 hours. This increases the number of T-suppressor lymphocytes. When antigen-specific cell fractions are desired, preferably 1-2 days prior to the first immunization the mammal receives one injection of anti-lymphocyte serum (0.5 ml/mouse i.p. or i.v.). This stimulates the T-suppressor cell population. When obtaining cell fractions specific to a particular antigen by immunising a mammal with the antigen two immunizations a week to 10 days apart are usually sufficient and the animals can be sacrificed 3-15 days after the second immunization.

When obtaining a cell fraction by first separating T-suppressor cells, the suppressor T lymphocyte population can be obtained by the following procedure:

The total lymphocyte population, suspended in tissue culture medium (e.g. 199 medium, Gibco, Glasgow) is incubated for 30-60 minutes at $37°C$ on a plastic surface (e.g. Falcon tissue culture flasks) as a thin layer. Typically the lymphocytes are suspended at $10^6$ per ml of tissue culture medium and the tissue culture flask is seeded with a layer of 1 mm in thickness in an atmosphere of 5% $CO_2$ in an incubator. At the end of the incubation period the cells non-adherent to the plastic are recovered. The adherent cells are macrophages and are discarded. The non-adherent cells are subsequently incubated on a plastic surface (Falcon flask) previously coated with an anti-gamma-globulin specific

- 8 -

for the species of mammal from which the lymphocytes were extracted.

The adherent cells, being B-lymphocytes, are discarded, whereas the non-adherent cells, being T-lymphocytes, are washed and incubated under the same conditions on a plastic surface coated with an anti-T suppressor lymphocyte antiserum, e.g. OKT-8 (Ortho Pharmaceuticals) for human T-suppressor lymphocytes or LYT 2.2 (Cedarlane Lab.,Ontario,Canada) for murine T-suppressor cells. The non-adherent cells are discarded whereas the adherent T-suppressor cells are recovered by washing the plastic surface with PBS (phosphate buffer saline) containing 5% by weight of lidocaine chlorhydrate at $4°C$.

In order to obtain the desired fraction from the T-suppressor cells, the cells thus recovered are washed and disrupted by repeated freezing-thawing techniques or by means of a homogeniser or sonication. The disrupted cells can then be dialysed through a dialysis bag (Visking cellulose) against distilled water or a vacuum. Alternatively, the disrupted cells can be introduced into an ultra-filtration apparatus possessing, for example, an Amicon filter. The dialysate or filtrate thus obtained comprises the suppressor factor and contains no inducer factor.

A yet further method of preparing a composition of the invention comprising suppressor factor specific to a given antigen is to obtain the suppressor factor from cells of lymphoid origin of a human or other mammal tolerant to the antigen in question, i.e. when challenged with the antigen no immune response to the antigen is mounted by the mammal's immune system. Tolerance to an antigen can be achieved by challenging either an embryo in utero or a mammal with a very small amount of antigen over a very long period of time. A mammal which is tolerant with respect to an antigen has no inducer factor specific to that antigen.  Compositions according to the invention comprising suppressor factor specific to the antigen can be prepared by the methods described above.

Compositions according to the invention contain substantially no molecules with a molecular weight of more than 12,000 from the cells of lymphoid origin from which the suppressor factor has been obtained.  This ensures that the compositions do not contain any viral genomes or any material which could be antigenic and thus elicit an anaphylactic reaction.

The suppressor factor may also be present in the milk of a mother or in urine, having been secreted into the body fluids from the cells of lymphoid origin. A composition according to the invention can therefore be prepared from the milk or urine by the techniques described above in relation to the cell fractions obtained from the cells of lymphoid origin.

The suppressor factor can be replicated by a lymphoblastoid cell line, e.g. as described in G.B. Patent Specification No. 1,443,948.  For example, a suppressor cell-containing cell fraction, e.g. dialysate, is filtered through a Milipore membrane (0.22 $\mu$m).  The filtrate is added to a culture of lymphoblastoid cells at a concentration of 5 x 10$^5$ cells/ml and at a 2:1 ratio (2 suppressor cell equivalents:1 lymphoblastoid cell). The cells thus induced are maintained in culture until a number sufficient for extraction of further fractions comprising suppressor factor is obtained, usually more than 10$^9$ cells and preferably more than 10$^{10}$ cells.

A preferred lymphoblastoid cell line for use in this replication procedure is the known LDV/7 cell line.  This cell line is described in G.B. Patent Specification No. 1,592,954.

- 10 -

A composition such as a cell fraction of the invention can be lyophilised, for example for storage. When required for use it can then be resuspended in a suitable volume of a physiologically acceptable diluent so as to form an isotonic solution.

The invention therefore also provides a pharmaceutical composition comprising as active ingredient a suppressor factor, together with a physiologically acceptable carrier or diluent and the composition containing substantially no inducer factor specific to any antigen or to an antigen to which the suppressor factor is specific, the suppressor factor having been obtained from cells of lymphoid origin from a human or other mammal and the composition containing substantially no molecules from said cells of lymphoid origin with a molecular weight of 12,000 or more. The composition may be suitable for oral administration or injection. It may be in the form of an injectable preparation, powder, tablets,capsules, gel, cream or eye lotion. Any suitable conventional carrier or diluent can be used.

A composition according to the present invention is useful in suppressing the immune response of a human or other mammal to an antigen. This is achieved according to the invention by administering to the human or other mammal an effective amount of a composition of the invention. The suppressor factor in the composition may be antigen non-specific or, where it is desired to suppress the immune response to a particular antigen, specific to that antigen,or both types may be present.

The composition can be used to treat allergic disorders in mammals, including humans. Diseases that can be treated include hay fever, allergic asthma, allergic conjunctivitis, allergic skin (eczema, urticaria) or lung disorders, and allergic food reactions. Thus according to the invention allergic manifestations in a

0105749

- 11 -

mammal are treated by administering to the mammal an effective amount of a composition according to the invention. To treat specific allergic disorders the composition comprises suppressor factor specific to a given antigen such as a pollen or house dust.

Furthermore according to the invention transplant rejections in a mammal are combated or prevented by administering to the mammal a composition according to the invention which is specific to a histo-compatibility gene product, preferably to all such products, of the transplant.

It is also possible to use compositions according to the invention to combat or prevent autoimmune diseases, for example in a human. A mammal is immunised with respect to the antigen causing the autoimmune disease. Where the autoimmune disease is caused by a virus, the mammal is immunised with that viral antigen. A composition containing the suppressor factor thus induced can then be prepared and administered to the patient suffering from the autoimmune disease.

The composition of the invention is typically administered orally, topically or by injection, for example subcutaneously, intravenously or intramuscularly. The amount administered will depend upon a variety of factors, including the existing treatment of the patient and the severity of the disorder. Typically, the composition can be administered at a dose obtained of from $5.10^7$ to $10^9$ lymphoid cells, e.g. T-suppressor lymphocytes, preferably from $5.10^8$, from daily to once a week. This is applicable regardless of the route of administration.

The following Examples illustrate the invention. The Reference Example illustrates the L.M.I. test. The M.I. obtained by this test is used to measure cell-mediated immunity. The present compositions produce an increase in migration, i.e. of the M.I., of at least 20% when

- 12 -

compared with the M.I. of a control L.M.I. assay in which the leucocytes used in the assay are incubated with the corresponding antigen but not with a composition comprising suppressor factor according to the invention. When the M.I. is 1 or more the result is considered negative in terms of cell-mediated immunity, in other words indicating that a particular antigen reaction is being suppressed or a tolerance to an antigen established. Preferably, therefore, compositions according to the invention have an M.I. of 1 or more when assayed in the L.M.I. test.

Reference Example

The L.M.I. test was carried out as follows. The test is described in relation to T-suppressor cell dialysates, but is equally applicable to other compositions in accordance with the invention.

20 ml of blood are collected in a heparinized tube, and the leucocytes separated by the addition of 20% Plasmagel (Roger Bellon, Paris). The cell pellet is recovered after centrifugation of the supernatant at 300G for 15 min., and is then washed in saline containing 0.2% heparin (100 i.u./ml). After a further centrifugation (300 G for 15 min) the pellet is resuspended in 199 medium (Gibco, Glasgow). After an additional centrifugation of 15 min at 100 G, to remove the platelets which remain in the supernatant, the cells are resuspended at $6 \times 10^7$/ml in 199 medium containing by volume 5% foetal calf serum (FCS). The T-suppressor cell dialysates are added to the cell suspension at 2 : 1 ratio (i.e. 1 leucocyte : dialysate from 2 T-suppressor cells), and incubated for 90 minutes at 37°C. These cells are then washed and resuspended in tissue culture medium. This cell suspension is then distributed into heparinized capillary tubes (1.1 mm internal diameter : Prosciences, Paris). After centrifugation for 5 min at 300 G the capillaries are cut at the cell interface and each tube is placed in

- 13 -

the chamber of a Sterilin migration plate (Polylabo-Paul Block, Strasbourg).

The chambers contain either medium alone or medium containing ABA-BSA, pollen or house dust (ABA-BSA 40 $\mu$g/0.1 ml, pollen and house dust 1/100 and 1/1000 V/V). Preliminary studies showed that this dilution gave the best results. At least three capillaries are used for each antigenic solution. After an incubation of 18 hours at 37°C on a horizontal support, the migration surfaces are projected onto drawing-paper using a photographic enlarger and three diameters of each surface measured. The Migration Index (M.I.) is then calculated using the formula:

$$\text{M.I.} = \frac{\text{Average of the diameters in presence of antigen}}{\text{Average of the diameters in absence of antigen}}$$

T-suppressor cell fractions are considered active when they produce an increase of migration of at least 20%.

ARSNAT denotes azobenzenearsonate-N-acetyl-L-tyrosine. This compound is used as immunising antigen and as a test antigen for skin tests. ABA-BSA denotes an azobenzenearsonate-bovine serum albumin conjugate. It is used instead of ARSNAT in the L.M.I. test.

Example 1 - The Production of Dialysates from Spleen cells

Ten Balb/c mice were immunized i.p. with ARSNAT. Each injection consisted of 0.5 ml of ARSNAT per mouse in 0.5 ml of Freunds Complete Adjuvant (FCA). On the fourth day the mice were sacrificed and a suspension of spleen cells prepared in MEM (Minimum Essential Medium) medium ($10^6$ cells/ml). 20 ml of the cell suspension was poured into a 75 cm$^2$ Falcon flask and incubated at 37°C for 3 hours. Following this incubation, the macrophages adhered to the plastic and the resulting cell suspension contained non-adherent cells, i.e. mainly lymphocytes. This cell suspension was then incubated in another Falcon

- 14 -

flask coated with LYT 2.2 antiserum (Cedarlane Lab.), which is a monoclonal anti-T-suppressor cell antiserum, for 1 hr at 37°C. The non-adherent cells were removed and put into a third Falcon flask coated with anti-FAB antiserum. After 1 hr incubation the non-adherent cells were removed. The latter constitute Fraction 1 which contains T-helper and null lymphocytes. The cells attached to plastic coated with anti-Fab antiserum consist mainly of B lymphocytes and monocytes and constitute Fraction 3. The cells attached to the plastic coated with the LYT 2.2 antiserum are T-suppressor cells and constitute Fraction 2, whereas the cells adhering spontaneously to the plastic flask are mainly macrophages and constitute Fraction 4.

The various cell Fractions were recovered and lysed with distilled water and then submitted to a cycle of ten freezing-thawings. They were then subsequently dialysed against a vacuum through a dialysis bag with a molecular weight cut-off of 12,000 and the dialysate recovered. The dialysates were tested in a L.M.I. test where fresh spleen lymphocytes from ARSNAT immunised mice were used in the presence of ABA-BSA.

The inhibition observed in the presence of antigen only was 0.65 (Migration Index : MI). The 4 different dialysates were added to investigate their enhancement or inhibition of this reaction. The results were as follows:

Fraction 1 - no effect    - M.I. 0.65
Fraction 2 - enhancement - M.I. 1.25
Fraction 3 - no effect    - M.I. 0.70
Fraction 4 - no effect    - M.I. 0.75

Fraction 2 is the dialysate, specific to ARSNAT, obtained from T-suppressor cells. It is only this Fraction which has an enhancing effect on the L.M.I. test reaction, demonstrating that it is the dialysate obtained from the T-suppressor cells which has suppressor activity.

- 15 -

Example 2: - Preparation of a cell dialysate specific
               to ARSNAT

Ten Balb/c mice were immunized intra-peritoneally (i.p.) with 500 μg of ARSNAT in 0.5 ml of FCA. Twenty one days later the mice were sacrificed, the spleens were removed and a lymphocyte suspension was prepared using a Ficoll Hypaque gradient. The lymphocytes were suspended in 199 medium and incubated on a plastic flask for 1 hour. The non-adherent cells were recovered and incubated for 1 hour on a plastic flask previously coated with rabbit anti-mouse gamma-globulin. The non-adherent cells (mainly T lymphocytes) were recovered and incubated for 1 hour on a plastic flask coated with monoclonal antibody LYT 2.2 (Cedarlane Lab.) which is specific for Balb/c mouse suppressor T lymphocytes. The adherent cells were recovered (suppressor T lymphocytes) and lysed by sonication and dialysed through a dialysis bag with a cut off at 12,000 M.W.

Example 3: - Preparation of a cell dialysate which is
               non-specific

8 Balb/c mice were sacrificed, the spleens were removed and the desired cell dialysate was prepared as in Example 2.

Example 4: - Replication of T-suppressor cell transfer
               factor

T-suppressor cell dialysates from $10^7$ cells, prepared as in Example 2, were added to $5.10^6$ LDV/7 cells. The culture was maintained until $2.10^9$ cells were obtained. A dialysate was prepared from these cells as in Example 2.

Example 5: - Skin Tests

T-suppressor cell dialysates prepared as in Examples 2, 3 and 4 were tested in vivo by injection into Balb/c mice. Four groups of five mice were used. Mice in group 1 were injected with saline whereas those in groups 2, 3 and 4 were immunized with ARSNAT (1 injection of 500 µg in 0.5 ml of FCA). 48 hours after the injections, skin tests were conducted in groups 2, 3 and 4 using 20 µg of ABA-BSA in 0.1 ml of saline. All mice showed positive skin tests. Mice of group 2 were injected with T-suppressor cell dialysate ($10^8$/mouse) prepared in Example 2, whereas mice of group 4 were injected with T-suppressor cell dialysates from LDV/7 cells prepared in Example 4. Three days later all groups were skin-tested with ARSNAT.

The results are shown in Table 1. They demonstrate that the T-suppressor cell dialysates of Examples 2 and 4 specific to ARSNAT suppress the immune response observed in group 3 after challenge of the mice with ARSNAT.

TABLE I

| Group | Antigen used for immunization | Mice injected with T-suppressor cell dialysate ($10^8$/mouse) | Antigen used for skin-test | Response |
|-------|-------------------------------|--------------------------------------------------------------|----------------------------|----------|
| 1 | Saline | no | ABA–BSA | – |
| 2 | ARSNAT | yes | ABA–BSA | – |
| 3 | ARSNAT | no | ABA–BSA | + |
| 4 | ARSNAT | *yes | ABA–BSA | – |

\* T-suppressor cell dialysate obtained from LDV/7 cells

0105749

- 18 -

Example 6

Three mice were immunized i.p. with 500 µg of ARSNAT with 0.5 ml FCA. Twenty one days later the animals were sacrificed and lymphocytes were obtained from their spleens. One aliquot (1) of the lymphocytes was incubated with T-suppressor cell dialysate (lymphocyte : dialysate ratio of 1:2) for 90 minutes. The cells were subsequently washed and tested in the L.M.I. test in the presence of ABA-BSA. The M.I. were compared with those obtained with the aliquot (2) containing the unincubated cells as shown on Table II below (mean values of 3 different experiments).

Table II

| | | M.I. in presence of ABA-BSA | |
|---|---|---|---|
| Aliquot 1 | 0.92 | 0.90 | 0.88 |
| Aliquot 2 | 0.72 | 0.69 | 0.75 |

Example 7: - Non specific increase of M.I. by T-suppressor cell dialysates

Lymphocytes from 3 mice and 2 blood donors were incubated with T-suppressor cell dialysate (ARSNAT specific) obtained in Example 2, for 90 minutes. The M.I. of the incubated cells was compared to those which were not incubated with the T-suppressor cell dialysate, in the presence of ABA-BSA and, as shown on Table III, T-suppressor cell dialysate incubation increases significantly the M.I. in the presence of ABA-BSA.

Table III

| Blood donor lymphocytes | M.I. after incubation with ARSNAT specific T-Suppressor cell dialysates in presence of ABA-BSA | M.I. in presence of ABA-BSA, no incubation with T-suppressor cell dialysates |
|---|---|---|
| 1 | 1.20 | 0.97 |
| 2 | 1.30 | 0.95 |
| Mouse lymphocytes | | |
| 1 | 1.35 | 0.85 |
| 2 | 1.15 | 0.89 |

- 19 -

Example 8

Ten Balb/c mice were each twice injected with 0.5 ml of a house dust antigen preparation (Laboratoires Des Stallergenes catalogue no.815, comprising 50% house dust and 50% dermatophagoides pteronyssinus). The injections were a week apart. The mice were sacrificed and a T-suppressor cell fraction containing substantially no molecules with a molecular weight greater than 12,000 was obtained following the procedure of Example 2.

The suppressor factor was replicated using the LDV/7 cell line. The T-suppressor cell fraction was added in an amount equivalent $10^7$ T-suppressor cells to $5 \times 10^6$ LDV/7 cells in a culture medium of RPMI 1640 medium containing by volume 10% foetal calf serum. The cells were cultured until $4 \times 10^9$ cells were obtained, disrupted and dialysed through a dialysis membrane having a molecular weight cut-off of 12,000 to obtain mouse T-suppressor cell dialysate specific to house dust.

Six human patients aged 30-45 years were treated for 2 months with the mouse T-suppressor cell dialysate specific to house dust. These patients had allergic conjunctivitis and allergic rhinitis and skin testing had shown their sensitization to the above mentioned antigen. The T-suppressor cell dialysate was given orally three times a week in the form of capsules each comprising a powder of dialysate from $2 \times 10^8$ cells and lactose (one capsule per patient per dose).

The efficacy of the treatment was evaluated by the clinical results : reduction of the allergic symptoms. An improvement was noticed in all patients. However, 15 days after the end of the therapy 2 of the 6 patients relapsed their previous clinical status. The improvement under treatment was progressive but quite noticeable within the first ten days.

- 20 -

Example 9

Three hay fever sufferers were treated with non-specific cell dialysates prepared in accordance with Example 3. Each patient was treated once a week for two months with an injection of dialysate obtained from $2 \times 10^8$ cells. Prior to treatment, the hay fever was very severe with accompanying rhinitis and conjunctivitis. After two weeks from the first administration the symptoms became less severe and improvement was maintained throughout the course of treatment. However, the patients relapsed at the end of the treatment and the hay fever resumed its previous severity.

Example 10

Ten Balb/c mice were each injected twice with 0.5 ml of a grass pollen antigen preparation (Stallergenes Retard Pollens, cinq graminees, 10000 PNU/ml, Laboratoires Des Stallergenes, France). The injections were a week apart. A T-suppressor cell fraction was obtained and replicated using the LDV/7 cell line following the procedure described in Example 8.

Four patients allergic to grass pollen were treated in the spring of 1983 with the T-suppressor cell dialysates (S-CDL) according to the invention comprising suppressor factor specific to grass pollen. The S-CDL was administered orally as capsules, every other day for one month. Each daily dose consisted of a capsule of a powder of dialysate from $2 \times 10^8$ cells and lactose. L.M.I. test data is shown in Table IV below.

The allergic symptoms of all four patients improved within five days of the first S-CDL administration and had disappeared by 10 days after beginning treatment. The improvement outlasted the administration and all patients remained relapse-free throughout the 1983 pollen season.

- 21 -

## TABLE IV

LMI TEST IN PRESENCE OF S-CDL SPECIFIC TO GRASS POLLEN
USING LEUKOCYTES FROM PATIENTS ALLERGIC TO POLLEN

| PATIENT NO. | 1 | 2 | 3 | 4 |
|---|---|---|---|---|
| M.I. $\dfrac{\text{LEUKOCYTES + POL.}}{\text{LEUKOCYTES}}$ | $0.70^{x}$ | 0.80 | 0.78 | 0.80 |
| M.I. $\dfrac{\text{LEUKOCYTES + S-CDL + POL.}}{\text{LEUKOCYTES + S-CDL}}$ | $1.38^{x}$ | 1.08 | 1.01 | 1.02 |

[x] Mean value of four different experiments S-CDL / Lymphocyte Ratio 1 : 1

### Example 11

S-CDL comprising suppressor factor specific to grass pollen and prepared in accordance with the procedure described in Example 10 was used to treat one eye of a human patient suffering from seasonal allergic conjunctivitis. The S-CDL was prepared in the form of a saline solution at a concentration of $5 \times 10^{7}$ LDV/7 cell equivalents per ml saline.

Three drops of the S-CDL saline solution were administered to the left eye of the patient every six hours. Three drops of a commercially available antihistaminic drug (Antistina-Privina, Ciba) were administered to the right eye of the patient also every six hours. There was no improvement in the conjunctivitis in the right eye. There was a net improvement in the conjunctivitis in the left eye : the conjunctivitis symptoms disappeared.

- 22 -

Example 12

2 x $10^6$ Lymphocytes from Black/6 mice were injected i.p. twice into each of thirty Balb/c mice. The injections were a week apart.  Twenty days after the first injection the Balb/c mice were sacrificed. A T-suppressor cell fraction (S-CDL) was obtained.

Four Balb/c mice (group 1) were given skin-grafts from Black/6 mice.  S-CDL equivalent to $10^7$ T-suppressor cells was injected each day for five days into each of the Balb/c mice.  The survival time of the grafts was observed.  The survival time of skin-grafts from Black/6 mice given to a control group of four Balb/c mice to which no S-CDL was administered was also observed.  The results are given in Table V.

Table V

| Group | Survival Time (Days) |
|-------|----------------------|
| 1 | $30 \pm 2$ |
| control | $13.5 \pm 2$ |

- 23 -

C L A I M S

1. A composition, suitable for use in suppressing the immune response of a human or animal to an antigen, which composition comprises a suppressor factor which is capable of inducing immunosuppression or immunoregulation of the immune response to an antigen in a human or animal but comprises substantially no inducer factor capable of inducing the ability to mount a cell-mediated immune response in a human or animal to any antigen or to an antigen to which the suppressor factor is specific, said suppressor factor having been derived from cells of lymphoid origin of a human or other mammal and the composition containing substantially no molecules from said cells of lymphoid origin with a molecular weight of 12,000 or more.

2. A composition according to claim 1 in which the suppressor factor is specific to a particular antigen and which contains substantially no inducer factor specific to that antigen.

3. A composition according to claim 1 in which the suppressor factor is antigen non-specific, having been obtained from cells of lymphoid origin extracted from a human or other mammal which has not been immunised with respect to a particular antigen.

4. A composition according to any one of the preceding claims which is in the form of a dialysate.

5. A composition according to any one of the preceding claims which has a Migration Index of 1 or more when assayed in the Leucocyte Migration Inhibition Test.

6. A pharmaceutical composition according to any one of the preceding claims which further comprises a physiologically acceptable carrier or diluent.

- 24 -

7. A process for the preparation of a composition suitable for use in suppressing the immune response of a human or animal to an antigen, which process comprises preparing from cells of lymphoid origin derived from a human or other mammal a composition comprising a suppressor factor which is capable of inducing immunosuppression or immunoregulation of the immune response to an antigen in a human or other mammal but containing substantially no inducer factor capable of inducing the ability to mount a cell-mediated immune response in a human or other mammal to any antigen or to an antigen to which the suppressor factor is specific and substantially no molecules from said cells of lymphoid origin with a molecular weight of 12,000 or more.

8. A process according to claim 7 in which said cells of lymphoid origin derived from a human or other mammal have been extracted from a human or other mammal.

9. A process according to claim 7 in which said cells of lymphoid origin derived from a human or other mammal have been obtained by extracting cells of lymphoid origin from a human or other mammal and culturing the extracted cells in vitro.

10. A process according to claim 8 or 9 in which the said human or other mammal has been immunised or is tolerant with respect to a particular antigen.

11. A process according to claim 7 in which said cells of lymphoid origin derived from a human or other mammal have been obtained by culturing cells of an established cell line of lymphoid origin in the presence of a said composition comprising suppressor factor but containing substantially no inducer factor and substantially no said molecules with a molecular weight of 12,000 or more.

- 25 -

12. A process according to claim 11 in which the said composition had been prepared by a process as claimed in any one of claims 8 to 10.

13. A process according to any one of claims 7 to 10 in which the said cells of lymphoid origin derived from a human or other mammal are T-suppressor cells and the said T-suppressor cells are disrupted and a cell fraction is prepared from the contents of the said T-suppressor cells which contains substantially no molecules with a molecular weight of 12,000 or more.

14. A process according to any one of claims 7 to 12 in which a cell fraction containing substantially no molecules with a molecular weight of 12,000 or more is prepared from the said cells of lymphoid origin derived from a human or other mammal and inducer factor is removed from the cell fraction thus obtained.

15. A process according to claim 14 in which substantially all inducer factor specific to a particular antigen is removed from the cell fraction thus obtained.

16. A process according to any one of claims 9, 11 or 12 in which the desired composition comprising suppressor factor but containing substantially no inducer factor and substantially no said molecules with a molecular weight of 12,000 or more is prepared from the medium in which the said culturing occurs.